# FASCICULE DE BREVET EUROPEEN

(11) **EP 2 238 236 B1**
(45) Date de publication et mention de la délivrance du brevet: **29.03.2017**
(21) Numéro de dépôt: 09715137.7
(22) Date de dépôt: 08.01.2009
(51) Int. Cl.: B01L 3/00, C12N 15/10, C12M 1/00

(54) **DISPOSITIF ET PROCÉDÉ POUR ISOLER ET CULTIVER DES CELLULES VIVANTES SUR FILTRE OU EXTRAIRE LEUR MATÉRIEL GÉNÉTIQUE**
VORRICHTUNG UND VERFAHREN ZUR ISOLIERUNG UND KULTIVIERUNG LEBENDER ZELLEN AUF EINEM FILTER ODER DERER EXTRAKTION VON GENETISCHEM MATERIAL
DEVICE AND METHOD FOR ISOLATING AND CULTIVATING LIVE CELLS ON A FILTER OR EXTRACTING THE GENETIC MATERIAL THEREOF

(30) Priorité: 09.01.2008 FR 0850117; 02.09.2008 FR 0855878
(43) Date de publication de la demande: 13.10.2010
(73) Titulaire: Screencell, 75013 Paris (FR)
(72) Inventeur: CAYRE, Yvon, 75013 Paris (FR); BENALI-FURET, Linda Naoul, 95200 Sarcelles (FR); AUCANT, Cécile, 95200 Sarcelles (FR); WECHSLER, Janine, 95200 Sarcelles (FR)
(74) Mandataire: Cornuejols, Georges
(86) Numéro de dépôt international: PCT/FR2009/000019
(87) Numéro de publication internationale: WO 2009/106760

(56) Documents cités:
- EP-A- 0 463 897
- EP-A- 0 503 128
- EP-A- 1 455 187
- WO-A-96/37301
- WO-A1-99/00193
- DE-A1- 19 820 178
- DE-B3-102005 008 220
- FR-A- 2 801 660

## Description

La présente invention concerne un dispositif et un procédé pour isoler et cultiver des cellules vivantes sur un filtre ou extraire du matériel génétique amplifié de cellules vivantes isolées sur un filtre. Elle s'applique, en particulier, à isoler et cultiver des cellules particulières présentes dans un liquide, notamment le sang ou à extraire le matériel génétique de ces cellules particulières.

Certaines cellules particulières du sang, par exemple les cellules tumorales ou les trophoblastes, sont en très faible proportion et doivent être décomptées pour une analyse cytologique. Cependant, par rapport au principal des autres cellules présentes dans le sang, elles présentent une plus grande dimension.

Il est connu d'appliquer un tampon de fixation à base de formaldéhyde à un échantillon sanguin pour fixer les cellules recherchées, puis de faire passer le liquide résultant dans un filtre poreux. Ce filtre est ensuite utilisé pour y examiner les cellules recherchées sous microscope, en laboratoire. Cependant, cette procédure ne permet pas l'obtention de cellules vivantes.

Or, l'inventeur a déterminé que l'obtention de cellules vivantes permettrait d'envisager l'identification de marqueurs spécifiques et d'appliquer dans de bonnes conditions des techniques de biologie moléculaire, de cytogénétique et de « FISH » (acronyme de « Fluorescence In Situ Hybridization », pour hybridation fluorescente in situ) pour le diagnostic d'anomalies génétiques dans des cellules tumorales ou trophoblastiques.

La présente invention vise à remédier à ces inconvénients et à répondre à ce besoin en permettant de recueillir, dans des conditions compatibles avec des examens de laboratoire de routine, des cellules vivantes pouvant être subséquemment cultivées dans des milieux adaptés en présence de facteurs de croissance adéquats.

La présente invention concerne aussi l'extraction du matériel génétique amplifié de cellules isolées sur filtre et la détection de mutations et des niveaux de l'expression de gènes de sensibilité et de résistance aux thérapies ciblées ou d'anomalies génétiques.

Elle s'applique, en particulier à recueillir et à amplifier de façon uniforme l'ADN ou l'ARN de cellules particulières présentes dans un liquide, notamment le sang.

Il est connu, par exemple du document WO200610036 ( PCT/FR 2006/000562), d'appliquer un tampon de fixation à base de formaldéhyde à un échantillon sanguin pour fixer les cellules recherchées, puis de faire passer le liquide résultant dans un filtre poreux. Ce filtre est ensuite analysé en laboratoire pour y rechercher les cellules sous microscope. On peut, par la suite, les prélever sur le filtre pour des analyses, par exemple par une analyse génétique.

Cependant, cette procédure ne peut faire l'objet d'une reproduction à grande échelle et à un coût raisonnable, du fait du temps, des matériels et de la précision de travail qu'elle implique. Or cette reproduction à grande échelle et moindre coût permettrait la conduite d'analyses de biologie moléculaire à la fois sur des cellules tumorales et sur des cellules trophoblastiques.

La présente invention vise aussi à remédier à ces inconvénients et à répondre à ce besoin en permettant de recueillir dans des conditions compatibles avec des examens de laboratoire de routine, une grande proportion du matériel cellulaire, en particulier, ARN et ADN, des cellules considérées, en bon état.

A cet effet, selon un premier aspect, la présente invention vise un dispositif pour isoler des cellules vivantes sur un filtre caractérisé en ce qu'il comporte :
- un support de filtre solidaire d'un filtre, le support de filtre prend la forme d'une rondelle,
- un compartiment présentant une ouverture supérieure et une ouverture inférieure adaptée à retenir ledit support et ledit filtre, et
- un moyen mobile par rapport audit compartiment pour appliquer une force sur le support et libérer ledit support, ledit moyen étant adapté à se déplacer parallèlement à l'axe du compartiment pour libérer le support de l'extrémité inférieure du compartiment.

On évite ainsi toute contamination ou détérioration des cellules vivantes sur le filtre ou du matériel génétique. Le contenu du compartiment est maintenu à l'état stérile lors des manipulations sous une hotte à flux laminaire adaptée. Ainsi, toutes les étapes pour isoler et cultiver les cellules ou pour extraire et analyser leur matériel génétique peuvent être effectuées dans des conditions stériles.

Ce dispositif permet ainsi la récupération des cellules vivantes d'intérêt dans des conditions parfaitement compatibles avec leur mise en culture pour poursuivre des examens de caractérisation cytomorphologique et de cytogénétique. Cette récupération est effectuée directement sur filtre et pratiquement sans perte de cellules recherchées. On recueille ainsi, à moindre coût, une grande proportion des cellules considérées, en bon état, dans des conditions compatibles avec une culture de routine en laboratoire.

Le dispositif objet de la présente demande permet aussi de recueillir du matériel cellulaire de cellules particulières, rapidement et efficacement, par exemple, après réalisation :
- d'une étape de filtration au cours de laquelle le principal du liquide et des dites autres cellules passe à travers un filtre dont les micropores ont un diamètre intermédiaire entre celui des dites cellules particulières et celui d'autres cellules,
- d'une étape de lyse et d'amplification de l'ADN et/ou de l'ARN dans ledit compartiment et
- d'une étape de récupération du matériel génétique amplifié des cellules lysées, sur le filtre.

Le dispositif objet de la présente demande comporte un embout amovible fixé de façon étanche et amovible et adapté à interdire le mouvement relatif du moyen mobile et dudit compartiment qui permet d'appliquer ladite force et libérer ledit support de filtre.

Grâce à ces dispositions, on garantit le maintien en position du porte-filtre. De plus, l'embout peut le protéger contre les éclaboussures et la contamination,

Ainsi, on retient le filtre pendant la filtration puis on le libère, pour sa récupération, en retirant l'embout et en mettant en mouvement respectif le moyen mobile et le compartiment pour appliquer la force qui libère le support de filtre.

Selon des caractéristiques particulières, ledit embout présente une ouverture inférieure qui s'adapte à une chambre de dépression d'un système d'aspiration.

Grâce à ces dispositions, on peut filtrer, par aspiration dans la chambre de dépression, les cellules plus petites que les cellules d'intérêt et les éventuelles cellules lysées et le principal du liquide présent dans le compartiment.

Selon des caractéristiques particulières, ledit support de filtre prend la forme d'une rondelle. On rappelle, ici, qu'une rondelle est un porte-filtre annulaire.

Selon des caractéristiques particulières, ledit support de filtre est en PVC. En effet, l'inventeur a détecté que cette matière ne flotte pas, à la différence du polyéthylène hydrophobe, et est plus rigide que ce dernier ce qui est favorable à l'utilisation du support de filtre en fond de puits de culture.

Selon des caractéristiques particulières, ledit support de filtre est en PVC rigide. Ces dispositions permettent la manipulation du filtre au cours d'analyses de cytomorphologie et de cytogénétique.

Selon des caractéristiques particulières, ledit support de filtre prend la forme d'une lamelle couvre objet.

Selon des caractéristiques particulières, ledit support de filtre à des dimensions supérieures ou égales à une lamelle couvre objet. Ces dispositions permettent le montage du filtre entre lame et lamelle.

Selon des caractéristiques particulières, ledit support de filtre possède une épaisseur inférieure ou égale à 0,4 mm et, préférentiellement, à 0,3 mm.

Grâce à ces dispositions, le support de filtre peut être utilisé sous microscope, même avec de forts grossissements, sans risquer de toucher une partie du microscope lors de déplacement latéraux du support de filtre.

Selon des caractéristiques particulières, ledit support de filtre prend la forme d'une partie, au moins, d'un tube Eppendorf.

Selon des caractéristiques particulières, ledit support de filtre reproduit, dans sa partie supérieure intérieure, la forme de la partie supérieure intérieure d'un tube Eppendorf.

Grâce à ces dispositions, on peut fermer l'ouverture supérieure dudit support de filtre avec un bouchon de tube Eppendorf.

Selon des caractéristiques particulières, ledit support de filtre reproduit, dans sa partie inférieure intérieure, la forme de la partie inférieure intérieure d'un tube Eppendorf.

Selon des caractéristiques particulières, ledit filtre possède un diamètre inférieur ou égal à 5 mm et, préférentiellement, à 3.5 mm ce qui favorise la lyse des cellules et la pré-amplification du matériel génétique.

On peut ainsi simuler une surface inférieure intérieure d'un tube Eppendorf.

Selon des caractéristiques particulières, ledit support de filtre reproduit, dans, sa partie supérieure extérieure, la forme de la partie supérieure intérieure d'un tube Eppendorf.

Grâce à ces dispositions, le support de filtre peut s'insérer dans la partie haute d'un tube Eppendorf.

On peut ainsi créer une étanchéité entre la partie supérieure extérieure de ledit support de filtre et la partie supérieure intérieure d'un tube Eppendorf.

Grâce aux trois dernières dispositions énoncées ci-dessus, le support de filtre présente une fonction colonne.

Selon des caractéristiques particulières, le support de filtre comporte du polycarbonate.

Grâce à ces dispositions, les pores du filtre retiennent le tampon de lyse et les réactifs pour amplifier le matériel génétique.

Selon des caractéristiques particulières, le support de filtre est mécaniquement lié audit compartiment jusqu'à l'application de ladite force.

Selon des caractéristiques particulières, ledit support de filtre est mécaniquement liée audit moyen mobile jusqu'à l'application de ladite force.

Selon un deuxième aspect, la présente invention vise un procédé pour isoler des cellules vivantes sur un filtre caractérisé en ce qu'il comporte :
- une étape de solidarisation d'un support de filtre solidaire d'un filtre, le support de filtre prenant la forme d'une rondelle, avec un compartiment présentant une ouverture supérieure et une ouverture inférieure ou avec un moyen mobile par rapport audit compartiment et
- une étape de mise en mouvement respectif dudit moyen mobile et dudit compartiment pour appliquer une force sur le support de filtre et libérer ledit support de filtre, ledit mouvement se faisant parallèlement à l'axe du compartiment pour appliquer une force sur le support de filtre et libérer ledit support de filtre de l'extrémité inférieure du compartiment.

Selon des caractéristiques particulières, au cours de l'étape de mise en mouvement, on fait passer directement le support de filtre à un puits de culture.

On évite ainsi toute contamination ou détérioration des cellules vivantes sur le filtre ou du matériel génétique extrait. Grâce à chacune de ces dispositions, le contenu du compartiment est maintenu à l'état stérile lors des manipulations sous une hotte à flux laminaire. Ainsi, toutes les étapes sont effectuées dans des conditions stériles adaptées à la culture cellulaire ou à l'extraction de matériel génétique.

Les avantages, buts et caractéristiques particulières de ce procédé étant similaires à ceux du dispositif objet de la présente invention, tel que succinctement exposé ci-dessus, ils ne sont pas rappelés ici.

Le procédé objet de la présente invention permet ainsi de recueillir du matériel cellulaire rapidement et efficacement. En général, le matériel cellulaire récupéré grâce à la mise en oeuvre de la présente invention est le matériel génétique des cellules. Le procédé objet de la présente invention permet ainsi la récupération, directement sur filtre et pratiquement sans perte, du matériel génétique de cellules rares, jusqu'à une seule cellule isolée. On recueille ainsi une grande proportion du matériel génétique des cellules considérées, en bon état, dans des conditions compatibles avec des examens de laboratoire de routine.

Le procédé objet de la présente demande comporte, à la suite de l'étape de lyse, une étape d'amplification de l'ADN et/ou de l'ARN et, au cours de l'étape de récupération, on récupère du matériel génétique amplifié des cellules lysées.

Selon des caractéristiques particulières, au cours de l'étape de lyse et d'amplification, on effectue une amplification uniforme conservant l'aspect quantitatif de l'ADN et de l'ARN.

Le procédé objet de la présente demande comporte, en outre, une étape de détection, au cours de laquelle l'ADN amplifié est utilisé comme matrice pour détecter au moins une mutation de gêne de sensibilité ou de résistance à au moins une thérapie ciblée.

Selon des caractéristiques particulières, le procédé objet de la présente demandé , tel que succinctement exposé ci-dessus, comporte, en outre, une étape de détection, au cours de laquelle l'ADN amplifié est utilisé comme matrice pour détecter une variation de niveau d'expression de gènes de sensibilité ou de résistance aux thérapies ciblées.

Selon des caractéristiques particulières, le procédé objet de la présente demandé , tel que succinctement exposé ci-dessus, comporte, en outre, une étape de détection au cours de laquelle on convertit de l'ARN en ADNc et on utilise ledit ADNc pour détecter le niveau d'expression de gène de sensibilité ou de résistance aux thérapies ciblées.

Selon des caractéristiques particulières, au cours de l'étape de détection, on met en oeuvre une PCR (acronyme de « polymerase chain reaction ») quantitative et en temps réel.

Selon des caractéristiques particulières, au cours de l'étape de détection, on met en oeuvre au moins un couple d'amorces sens et anti-sens pour amplifier une séquence d'intérêt prédéterminé.

Selon des caractéristiques particulières, au cours de l'étape de détection, on met en oeuvre au moins un couple de sondes.

Selon des caractéristiques particulières, au moins deux sondes d'un couple de sondes sont couplées à deux fluorochromes différents et sont définis pour que l'une reconnaisse la séquence mutée et que l'autre reconnaisse la séquence normale.

Selon des caractéristiques particulières, au moins un couple d'amorces et un couple de sondes sont adaptés à détecter la mutation G12D du gène K-ras de résistance à l'Erlotinib et au Gefitinib.

Selon des caractéristiques particulières, au moins une amorce comporte au moins 80 % de la séquence AGGCCTGCTGAAAATGACTGAATAT.

Selon des caractéristiques particulières, au moins une amorce comporte au moins 80 % de la séquence TCGTCCACAAAATGATTCTGAATTAGCT.

Selon des caractéristiques particulières, au moins une sonde comporte au moins 80 % de la séquence TTGGAGCTGGTGGCGT.

Selon des caractéristiques particulières, au moins une sonde comporte au moins 80 % de la séquence TGGAGCTGATGGCGT.

Selon des caractéristiques particulières, au moins un couple d'amorces et un couple de sondes sont adaptés à détecter la mutation G12V du gène K-ras de résistance à l'Erlotinib et au Gefitinib.

Selon des caractéristiques particulières, au moins une amorce comporte au moins 80 % de la séquence AGGCCTGCTGAAAATGACTGAATAT.

Selon des caractéristiques particulières, au moins une amorce comporte au moins 80 % de la séquence TCGTCCACAAAATGATTCTGAATTAGCT.

Selon des caractéristiques particulières, au moins une sonde comporte au moins 80 % de la séquence TTGGAGCTGGTGGCGT.

Selon des caractéristiques particulières, au moins une sonde comporte au moins 80 % de la séquence TTGGAGCTGTTGGCGT.

Selon des caractéristiques particulières, au moins un couple d'amorces et un couple de sondes sont adaptés à détecter la mutation G13C du gène K-ras de résistance à l'Erlotinib et au Gefitinib.

Selon des caractéristiques particulières, au moins une amorce comporte au moins 80 % de la séquence AGGCCTGCTGAAAATGACTGAATAT.

Selon des caractéristiques particulières, au moins une amorce comporte au moins 80 % de la séquence TCGTCCACAAAATGATTCTGAATTAGCT.

Selon des caractéristiques particulières, au moins une sonde comporte au moins 80 % de la séquence TTGGAGCTGGTGGCGT.

Selon des caractéristiques particulières, au moins une sonde comporte au moins 80 % de la séquence TTGGAGCTGGTTGCGT.

Selon des caractéristiques particulières, au moins un couple d'amorces et un couple de sondes sont adaptés à détecter la mutation L858R du gène EGFR de sensibilité augmentée à l'Erlotinib et au Gefitinib.

Selon des caractéristiques particulières, au moins une amorce comporte au moins 80 % de la séquence GCAGCATGTCAAGATCACAGATTT.

Selon des caractéristiques particulières, au moins une amorce comporte au moins 80 % de la séquence CCTCCTTCTGCATGGTATTCTTTCT.

Selon des caractéristiques particulières, au moins une sonde comporte au moins 80 % de la séquence CAGTTTGGCCAGCCCA.

Selon des caractéristiques particulières, au moins une sonde comporte au moins 80 % de la séquence CAGTTTGGCCCGCCCA.

Selon un troisième aspect, la présente demande vise un dispositif pour isoler des cellules vivantes sur un filtre, caractérisé en ce qu'il comporte :
- un support de filtre solidaire d'un filtre
- un compartiment présentant une ouverture supérieure et une ouverture inférieure adaptée à retenir ledit support et ledit filtre et
- un moyen d'application d'une force sur le support depuis l'intérieur du compartiment.

Ce dispositif permet la récupération des cellules vivantes d'intérêt dans des conditions parfaitement compatibles avec leur mise en culture pour poursuivre des examens de génétique.

Selon des caractéristiques particulières, le dispositif objet de la présente demande tel que succinctement exposé ci-dessus comporte, dans le compartiment, une pièce mobile adaptée à appuyer sur le support de filtre et à recevoir une force exercée par un piston inséré dans le compartiment.

Selon des caractéristiques particulières, le dispositif objet de la présente demande tel que succinctement exposé ci-dessus comporte, sur son ouverture inférieure, un embout retenant le filtre et le support de filtre, ledit embout étant fixé de façon étanche et amovible au compartiment.

Grâce à ces dispositions, on retient le filtre pendant la filtration puis on le libère, pour sa récupération, en retirant l'embout et en appliquant une force sur le support de filtre depuis l'intérieur du compartiment.

Selon des caractéristiques particulières, ledit embout présente une ouverture inférieure qui s'adapte à une chambre de dépression.

Grâce à ces dispositions, on peut filtrer, par aspiration dans la chambre de dépression, les cellules plus petites que les cellules d'intérêt et les éventuelles cellules lysées et le principal du liquide présent dans le compartiment.

Selon un quatrième aspect, la présente demande vise un procédé pour recueillir des cellules présentes dans un liquide, caractérisé en ce qu'il comporte :
- une étape de solidarisation d'un support de filtre à un compartiment présentant une ouverture supérieure et une ouverture inférieure adaptée à retenir à l'intérieur du dit compartiment ledit support et ledit filtre,
- une étape de filtration d'un liquide comportant des cellules vivantes, à travers ledit filtre et
- une étape d'application d'une force sur le support depuis l'intérieur du compartiment pour récupérer le filtre en dehors du compartiment.

Les avantages, buts et caractéristiques particulières de ce procédé étant similaires à ceux du dispositif objet du troisième aspect de la présente invention, tel que succinctement exposé ci-dessus, ils ne sont pas rappelés ici.

Selon un cinquième aspect, la présente demande vise un procédé pour isoler des cellules vivantes sur un filtre, caractérisé en ce qu'il comporte :
- une étape d'insertion d'un liquide comportant lesdites cellules et d'un tampon de filtration sans fixateur dans un compartiment, par l'intermédiaire d'une ouverture supérieure du compartiment, ledit compartiment présentant une ouverture inférieure, entre les deux ouvertures étant positionné un filtre dont les micropores ont un diamètre intermédiaire entre celui des dites cellules particulières et celui d'autres cellules,
- une étape de filtration au cours de laquelle le principal du liquide et lesdites autres cellules passent à travers le filtre et
- une étape de récupération du filtre et des cellules restées sur le filtre.

Ce procédé permet la récupération des cellules vivantes d'intérêt dans des conditions parfaitement compatibles avec leur mise en culture pour poursuivre des examens de génétiques. Cette récupération est effectuée directement sur filtre et pratiquement sans perte de cellules recherchées. On recueille ainsi une grande proportion des cellules considérées, en bon état, dans des conditions compatibles avec une culture de routine en laboratoire.

Selon des caractéristiques particulières, le procédé tel que succinctement exposé ci-dessus comporte, préliminairement à l'étape de filtration, une étape d'application d'un tampon de filtration sans fixateur constitué d'un milieu de culture et d'un agent lytique au liquide contenant les cellules particulières.

Selon des caractéristiques particulières, au cours de l'étape de filtration, on applique une aspiration par une dépression inférieure ou égale à 25 mBar en dessous du filtre.

Grâce à ces dispositions, la filtration est efficace et rapide et n'endommage pas les cellules vivantes à récupérer sur le filtre.

Selon des caractéristiques particulières, au cours de l'étape de filtration, un embout du compartiment retient le filtre dans le compartiment et, au cours de l'étape de récupération du filtre, on retire ledit embout du compartiment, on introduit dans le compartiment un piston et on applique avec ledit piston une force sur un support dudit filtre.

Cette force permet de faire sortir le support portant le filtre à l'extrémité inférieure du compartiment, et donc le filtre, du compartiment. De plus, la force ne s'exerçant pas directement sur le filtre et ne provoquant pas de surpression, les cellules portées par ce filtre ne sont pas endommagées lors de la récupération du filtre.

Selon des caractéristiques particulières, au cours de l'étape de récupération, on fait passer directement le filtre depuis le compartiment à un puits de plaque et/ou un flacon de culture.

On évite ainsi toute contamination ou détérioration des cellules vivantes sur le filtre. Grâce à chacune de ces dispositions, le contenu du compartiment est maintenu à l'état stérile lors des manipulations sous une hotte à flux laminaire tel que permettant la manipulation dans des conditions stériles des cellules. Ainsi, toutes les étapes sont effectuées dans des conditions stériles adaptées à la culture cellulaire.

Selon un sixième aspect, la présente demande vise un dispositif pour recueillir des cellules présentes dans un liquide, caractérisé en ce qu'il comporte :
- un moyen d'insertion du liquide et d'un tampon de filtration sans fixateur dans un compartiment, par l'intermédiaire d'une ouverture supérieure du compartiment, ledit compartiment présentant une ouverture inférieure,
- un filtre positionné entre les deux ouvertures du compartiment, ledit filtre ayant des micropores d'un diamètre intermédiaire entre celui des dites cellules particulières et celui d'autres cellules,
- un moyen de filtration pour faire passer, à travers un filtre, le principal du liquide et lesdites autres cellules et
- un moyen de récupération du filtre et des cellules restées sur le filtre.

Les avantages, buts et caractéristiques particulières de ce dispositif étant similaires à ceux du procédé objet du cinquième aspect de la présente invention, tel que succinctement exposé ci-dessus, ils ne sont pas rappelés ici.

D'autres avantages, buts et caractéristiques de la présente invention ressortiront de la description qui va suivre, faite, dans un but explicatif et nullement limitatif, en regard des dessins annexés, dans lesquels :
- la figure 1 représente, schématiquement et en perspective, l'assemblage des pièces d'un premier mode de réalisation du dispositif objet de la présente invention,
- les figures 2A à 2D représentent, schématiquement et en sections axiales perpendiculaires entre elles, le premier mode de réalisation du dispositif avant utilisation,
- les figures 3A à 3O représentent, schématiquement, en élévation ou en coupe, des étapes de mise en oeuvre du premier mode de réalisation du dispositif objet de la présente invention,
- la figure 4 représente, sous forme d'un logigramme, des étapes mises en oeuvre dans un premier mode de réalisation particulier du procédé objet de la présente invention,
- la figure 5 représente, schématiquement et en perspective, l'assemblage des pièces d'un deuxième mode de réalisation particulier du dispositif objet de la présente invention,
- les figures 6A à 6D représentent, schématiquement et en sections axiales perpendiculaires entre elles, le deuxième mode de réalisation du dispositif avant utilisation,
- les figures 7A à 7L représentent, schématiquement, en élévation ou en coupe, des étapes de mise en oeuvre du deuxième mode de réalisation du dispositif objet de la présente invention,
- la figure 8 représente, en coupe, un support de filtre intégré au deuxième mode de réalisation du dispositif objet de la présente invention et
- la figure 9 représente, sous forme d'un logigramme, des étapes mises en oeuvre dans un deuxième mode de réalisation particulier du procédé objet de la présente invention.

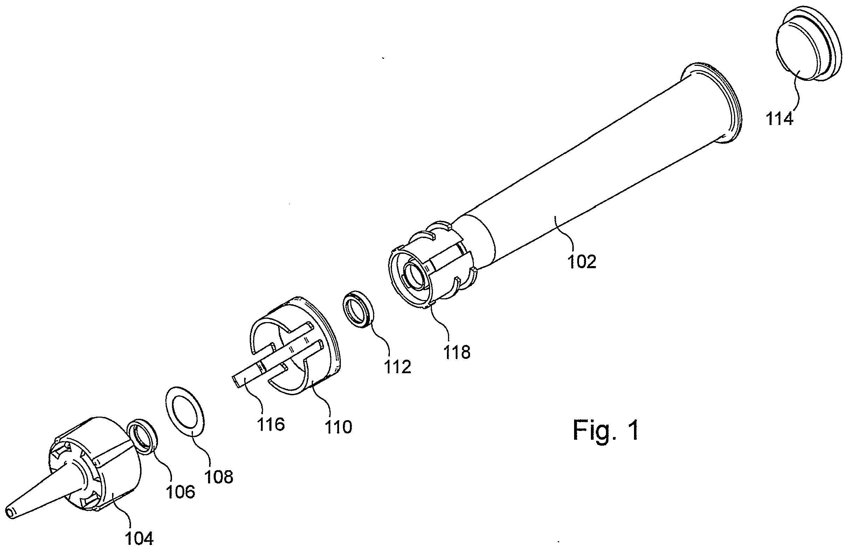

On observe, en figure 1, un réservoir, ou compartiment, 102, un embout 104, un joint 106, un support de filtre 108, un moyen mobile 110, un joint 112 et un bouchon 114.

Le compartiment 102 est de forme générale cylindrique. Son extrémité supérieure peut être obturée, de manière étanche, par le bouchon 114. L'extrémité inférieure du compartiment 102 présente, sur sa face externe, des anneaux discontinus dont les discontinuités guident des pattes du moyen mobile 110, lesdits anneaux guidant le corps du moyen mobile 110.

Ce moyen mobile 110 présente une forme générale cylindrique munie de deux pattes 116 s'étendant vers l'embout 104 et se resserrant dans cette direction de manière à être séparées, entre elles, d'une distance inférieure au diamètre du support de filtre 108. Comme on le verra, par la suite, cette forme particulière, notamment, celle des pattes 116 recourbées l'une vers l'autre, permet au moyen mobile 110, après retrait de l'embout 104, de pousser le support de filtre 108 afin de le libérer du compartiment 102, lors du mouvement du moyen mobile vers le support de filtre 108.

L'extrémité des pattes 116 du moyen mobile 110 et l'extrémité inférieure du compartiment 102 sont adaptés à pénétrer dans une boîte ou puits, de culture. En revanche, l'anneau discontinu extrême du compartiment 102 possède un diamètre adapté à ce qu'il prenne appui sur le bord de la boîte, ou puits, de culture.

A l'ouverture inférieure du compartiment 102 est placé, de façon étanche, stérile et amovible, un embout, ou adaptateur, 104 qui enserre la paroi extérieure du compartiment 102 et présente une ouverture inférieure effilée de plus petit diamètre que celui du compartiment 102.

Cette ouverture inférieure effilée de l'embout, ou adaptateur, 104 présente une longueur suffisante pour éviter de potentielles contaminations de l'extrémité du compartiment 102 par des éclaboussures provenant d'une chambre dans laquelle est réalisée la dépression.

L'embout 104 présente, de manière coordonnée avec la forme de l'extrémité inférieure du compartiment 102, qui possède des ergots latéraux 118, des moyens de blocage par rotation, pour enserrer lesdits ergots, de manière connue en soi. L'embout 104 garantit ainsi le maintien en position du porte-filtre pendant les étapes de filtration. De plus, l'embout 104 protège le filtre contre les éclaboussures et les contaminations.

L'extrémité inférieure du compartiment 102 présente une ouverture débouchant, après montage, sur le filtre porté par le support de filtre 108, lui-même retenu en position, d'une part, par l'extrémité inférieure du compartiment 102 et, d'autre part, par l'embout 104.

Le support de filtre 108 prend, dans le premier mode de réalisation, la forme d'une rondelle de forme annulaire. Le filtre est micro perforé et soudé sous le support de filtre 108 puis inséré avec elle dans l'extrémité inférieure du compartiment 102.

Le support de filtre 108 est, préférentiellement, en PVC et possède une épaisseur inférieure ou égale à 0,4 mm, et préférentiellement, inférieure à 0,3 mm. Son diamètre extérieur est, par exemple, de 12,6 mm. Le diamètre du filtre porté par le support de filtre 108 est, par exemple, de 8,2 mm.

Le compartiment 102, l'embout 104 et le moyen mobile 110 sont réalisés, par exemple, en polypropylène. Les joints 106 et 112 sont, par exemple, en silicone.

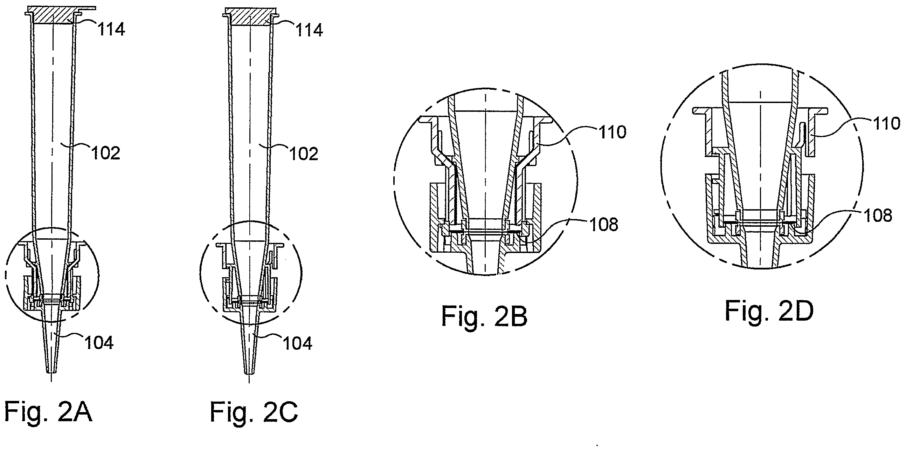

Les figures 2A et 2C sont des sections axiales, perpendiculaires entre elles, du premier mode de réalisation du dispositif objet de la présente invention, une fois assemblées les pièces illustrées en figure 1. Les figures 2B et 2D sont des vues de détails agrandies de parties des figures 2A et 2C, respectivement. On retrouve, dans les figures 2A à 2D, les éléments décrits en regard de la figure 1.

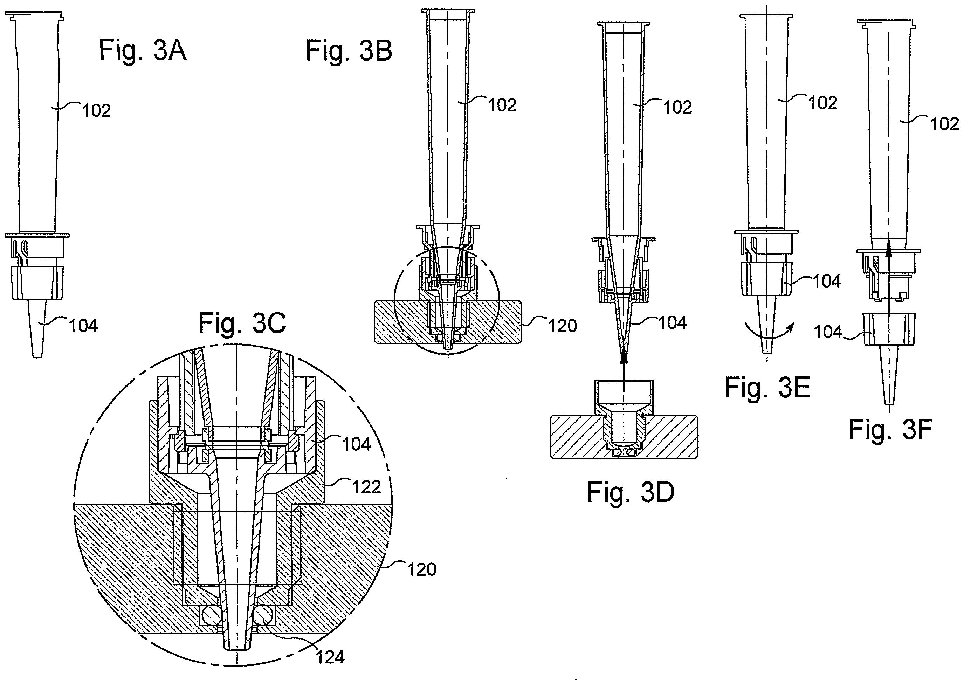

La figure 3A représente le dispositif en élévation, dans sa configuration de stockage. La figure 3B représente la mise en place du dispositif dans une platine 120 d'un système d'aspiration de type connu (non représenté) après retrait du bouchon 114 et introduction de liquide (non représenté), par exemple du sang, dans le compartiment 102, à travers son ouverture supérieure. La figure 3C est une vue de détails agrandie d'une partie de la figure 3B. On observe, en figures 3B et 3C, que l'embout 104 est maintenu en position dans une pièce 122 et qu'un joint torique 124 assure l'étanchéité de la liaison entre l'intérieur de l'embout et donc, à travers le filtre 108, l'intérieur du compartiment 102 et la chambre d'aspiration du système d'aspiration. On observe que l'embout 104 dépasse à l'intérieur de la chambre d'aspiration.

Lors de l'aspiration, certaines cellules particulières du liquide présent dans le compartiment 102, de plus fort diamètre, sont retenues par le filtre alors que le principal du liquide, du contenu et de la paroi des cellules lysées et les cellules de plus petites dimensions que les cellules à recueillir sont aspirés en dehors du compartiment 102, à travers le filtre.

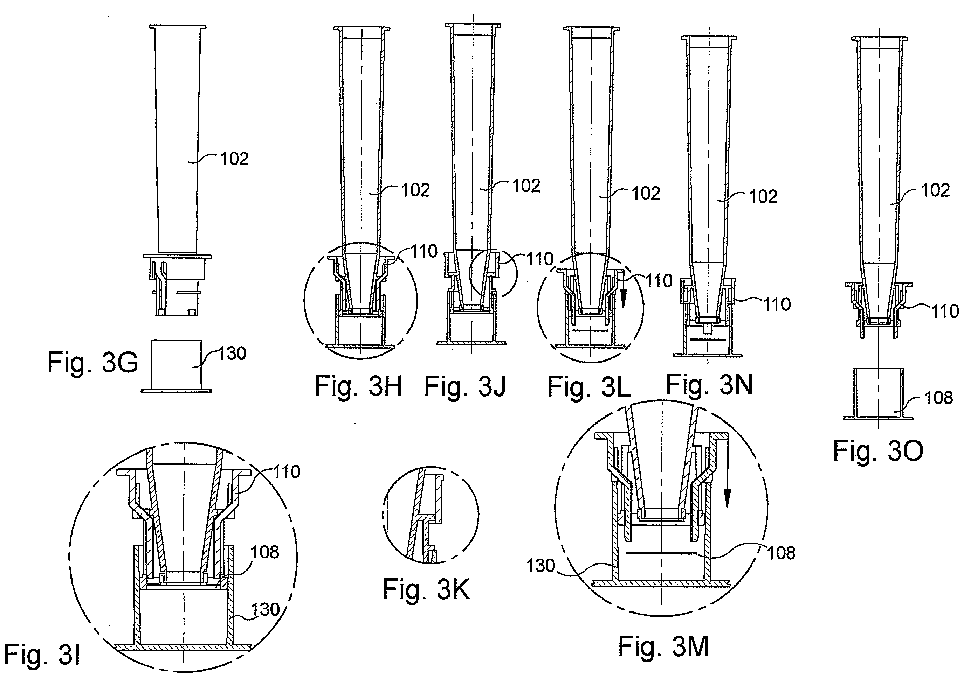

Comme illustré en figure 3D, après filtration, on retire le dispositif de la platine. Puis, comme illustré en figure 3E et 3F, on retire l'embout 104 après une rotation le libérant des ergots 118. Puis, comme illustré en figures 3G et 3H, on insère l'extrémité du compartiment 102 dans une boîte, ou un puits, de culture 130.

Comme exposé ci-dessus et comme illustré en figure 3I, l'extrémité rapprochée des pattes 116 du moyen mobile 110 et l'extrémité inférieure du compartiment 102 sont adaptés à pénétrer dans une boîte ou puits, de culture. En revanche, l'anneau discontinu extrême du compartiment 102 possède un diamètre adapté à ce qu'il prenne appui sur le bord de la boîte, ou puits, de culture 130.

Plus précisément, comme illustré en figures 3J et 3K, le moyen mobile 110 peut encore, dans cette position, bouger parallèlement à l'axe du compartiment 102.

Comme illustré en figures 3N et 3M, lors de ce mouvement, les pattes 116 du moyen mobile mis en mouvement par les doigts d'un opérateur, exercent une force verticale, de haut en bas, sur le support de filtre 108 et le libère de l'extrémité inférieure du compartiment 102. Le support de filtre 108 tombe alors dans la boîte, ou puits, de culture 130.

Enfin, comme illustré en figure 3O, on retire le compartiment 102 et le moyen mobile 110 de la boîte, ou puits, de culture 130.

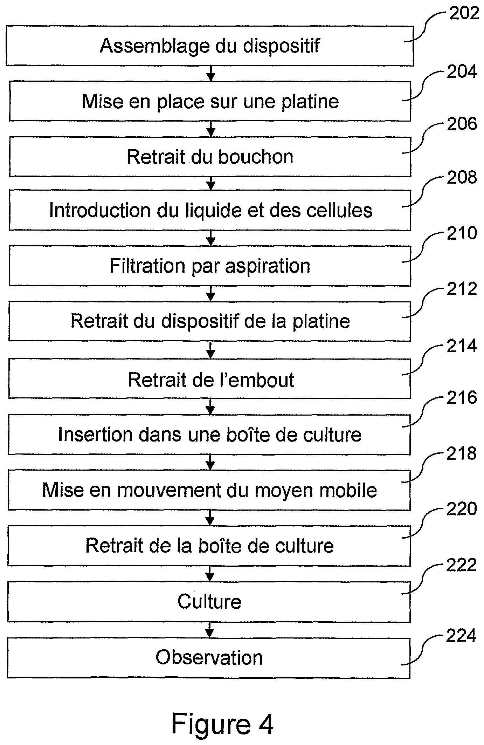

La figure 4 récapitule les étapes ainsi mises en oeuvre.

Au cours d'une étape 202, on assemble les pièces du dispositif. Au cours d'une étape 204, on met en place le dispositif dans une platine 120 d'un système d'aspiration. Au cours d'une étape 206, on retire le bouchon 114. Au cours d'une étape 208, on introduit un liquide, par exemple, du sang, contenant des cellules à cultiver, par l'extrémité supérieure du compartiment 102.

Au cours d'une étape 210, on effectue une filtration, par aspiration dans la chambre de dépression du système d'aspiration, des cellules plus petites que les cellules d'intérêt et les éventuelles cellules lysées et le principal du liquide présent dans le compartiment 102.

Au cours d'une étape 212, on retire le dispositif de la platine. Au cours d'une étape 214, on retire l'embout 104. Au cours d'une étape 216, on insère l'extrémité du compartiment 102 dans une boîte, ou un puits, de culture.

Au cours d'une étape 218, on met en mouvement respectif le moyen mobile et le compartiment pour exercer une force sur le support de filtre et le libérer afin qu'il tombe dans la boîte, ou puits, de culture. Au cours d'une étape 220, on retire le compartiment 102 et le moyen mobile 110 de la boîte, ou puits, de culture 130.

Au cours d'une étape 220, la culture est réalisée, de manière connue, dans le puits de culture 130. On observe que la présence du support 108 autour de la face supérieure du filtre, face qui porte les cellules isolées sur filtre, permet d'éviter que ces cellules ne quittent le filtre.

Lors de l'étape de mise en culture des cellules vivantes d'intérêt sur le filtre, le filtre est, par exemple, recouvert d'une mince couche de Matrigel (ou mis en contact avec une couche de Matrigel, marque déposée, préalablement mis au fond du puits de plaque et/ou du flacon de culture et sur laquelle il repose) contenant des facteurs adaptés à la croissance des cellules d'intérêt.

Lorsque l'on souhaite observer les cellules, ou leur matériel génétique, au cours d'une étape 222, on attrape le support de filtre 108, dont la saisie avec une brucelle est facilitée par la présence de trous cylindriques latéraux, ou encoches, dans la face supérieure du support de filtre 108.

Puis, on peut poser le support de filtre 108 sur une lame de verre et on appliquer, sur le filtre, une lamelle de verre en forme de disque de diamètre adapté à la surface supérieure libre du filtre. L'analyse des cellules ou de leur matériel génétique est, ensuite, effectuée de manière connue en soi.

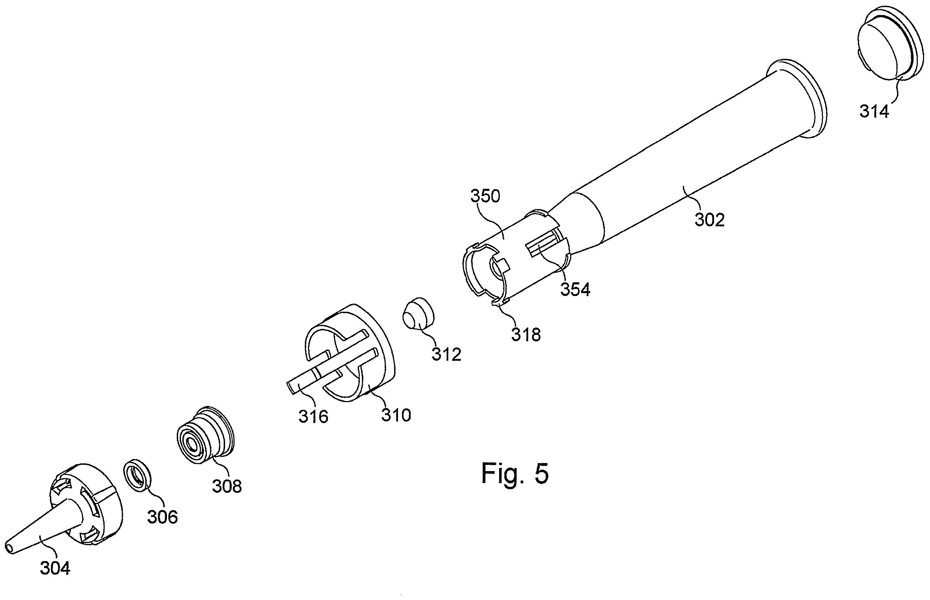

On observe, en figure 5, un réservoir, ou compartiment, 302, un embout 304, un joint 306, un support de filtre 308, un moyen mobile 310, un joint 312 et un bouchon 314.

Le compartiment 302 est de forme générale cylindrique. Son extrémité supérieure peut être obturée, de manière étanche, par le bouchon 314. L'extrémité inférieure du compartiment 302 présente, sur sa face externe, un cylindre 350 séparé du corps du compartiment 302, hormis par des liaisons mécaniques en forme de nervures latérales 352. Ce cylindre 350 possède un diamètre extérieur qui correspond au diamètre intérieur du corps du moyen mobile 310, afin de le guider en déplacement. Le cylindre 350 est muni de lumières 354 adaptées à laisser pénétrer et coulisser longitudinalement les pattes 316 du moyen mobile 310.

Le moyen mobile 310 présente une forme générale cylindrique munie de deux pattes 316 s'étendant vers l'embout 304 et se resserrant dans cette direction de manière à être séparées, entre elles, d'une distance inférieure au diamètre du support de filtre 308. Comme on le verra, par la suite, cette forme particulière, notamment, celle des pattes 316 recourbées l'une vers l'autre, permet au moyen mobile 310, après retrait de l'embout 304, de pousser le support de filtre 308 afin de le libérer du compartiment 302, lors du mouvement du moyen mobile vers le support de filtre 308.

L'embout 304 présente, de manière coordonnée avec la forme de l'extrémité inférieure du compartiment 302, qui possède des ergots latéraux 318, des moyens de blocage par rotation, pour enserrer lesdits ergots, de manière connue en soi. L'embout 304 garantit ainsi le maintien en position du porte-filtre pendant les étapes de filtration. De plus, l'embout 304 protège le filtre contre les éclaboussures et la contamination.

L'extrémité inférieure du compartiment 302 présente une ouverture débouchant, après montage, sur le filtre porté par le support de filtre 308, lui-même retenu en position, d'une part, par l'extrémité inférieure du compartiment 302 et, d'autre part, par l'embout 304.

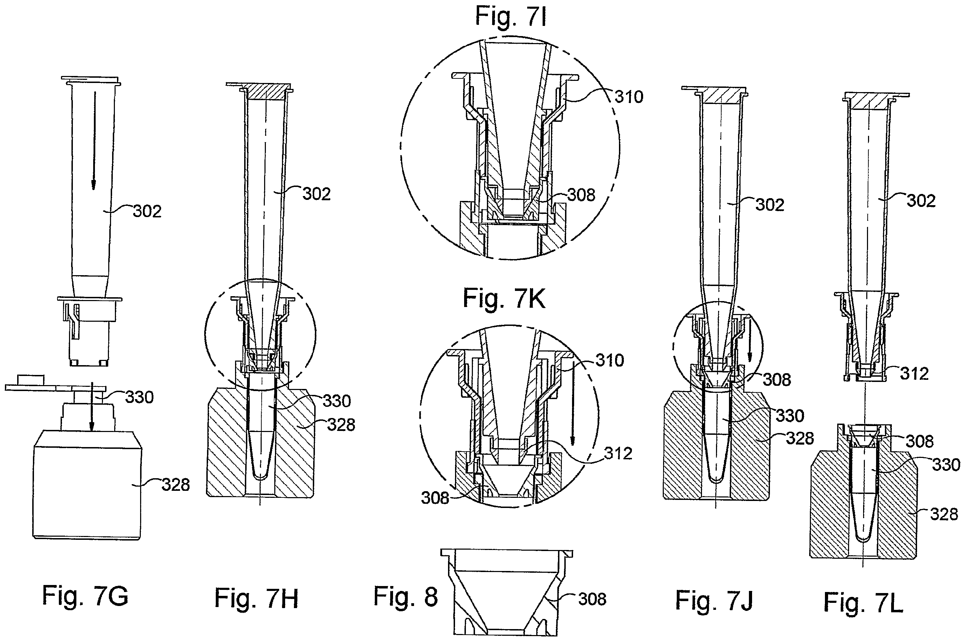

Comme illustré en figure 8, le support de filtre 308 prend, dans le deuxième mode de réalisation, une forme coordonnée à celle d'un tube Eppendorf.

En particulier :
- le support 308 présente, dans sa partie supérieure intérieure, la forme de la partie supérieure intérieure d'un tube Eppendorf, ce qui permet de fermer l'ouverture supérieure dudit support avec un bouchon de tube Eppendorf,
- le support 308 présente, dans sa partie inférieure intérieure, la forme de la partie inférieure intérieure d'un tube Eppendorf, ce qui permet de simuler une surface inférieure intérieure d'un tube Eppendorf et
- le support 308 présente, dans sa partie inférieure extérieure, la forme de la partie supérieure intérieure d'un tube Eppendorf, ce qui permet au support de filtre 308 de s'insérer dans la partie haute d'un tube Eppendorf.

De plus, le support de filtre 308 présente une fonction colonne pour permettre la lyse des cellules retenues sur filtre et le transfert du lysat cellulaire et du matériel génétique du support de filtre vers le tube Eppendorf.

Le support de filtre 308 est, préférentiellement, en PC (polycarbonate). Le compartiment 302, l'embout 304 et le moyen mobile 310 sont réalisés, par exemple, en polypropylène. Les joints 306 et 312 sont, par exemple, en silicone.

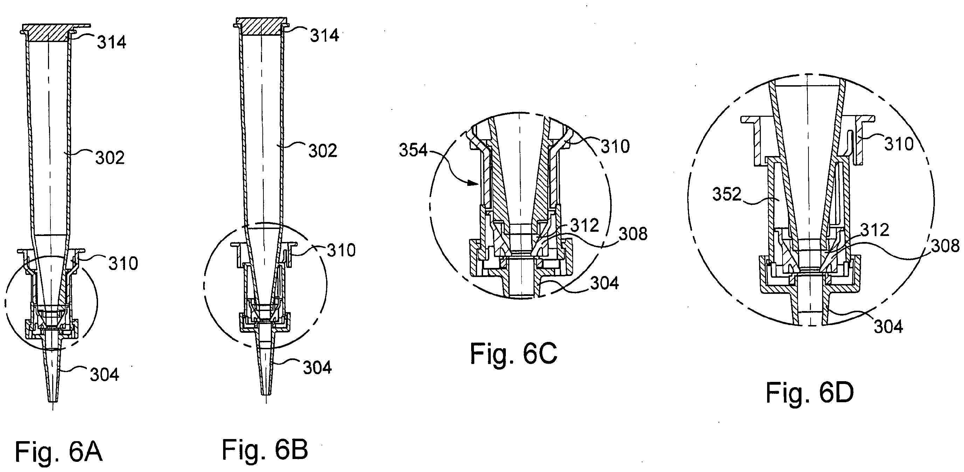

Les figures 6A et 6C sont des sections axiales, perpendiculaires entre elles, du premier mode de réalisation du dispositif objet de la présente invention, une fois assemblées les pièces illustrées en figure 5. Les figures 6B et 6D sont des vues de détails agrandies de parties des figures 6A et 6C, respectivement. On retrouve, dans les figures 6A à 6D, les éléments décrits en regard de la figure 5.

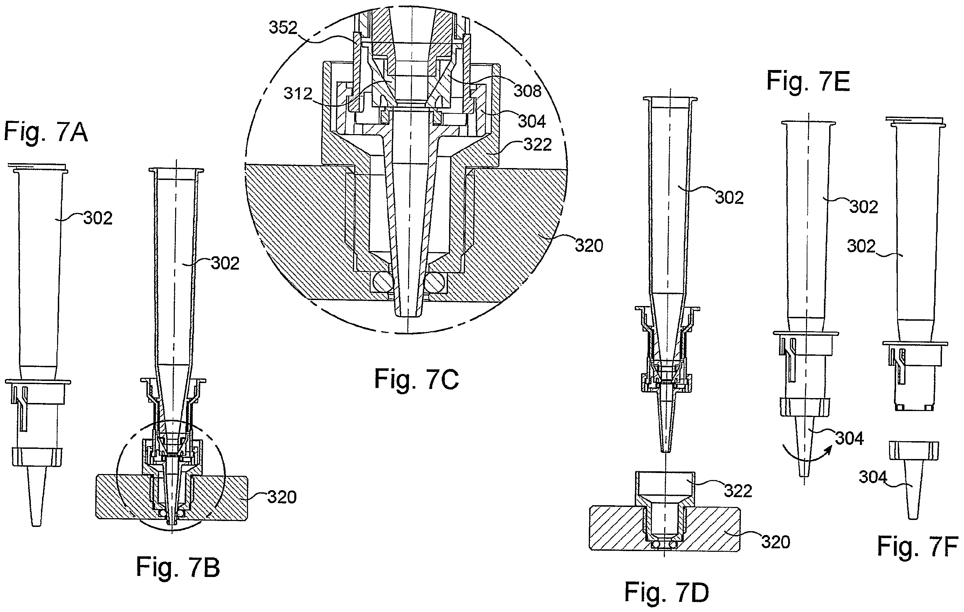

La figure 7A représente le dispositif en élévation, dans sa configuration de stockage. La figure 7B représente la mise en place du dispositif dans une platine 320 d'un système d'aspiration de type connu (non représenté) après retrait du bouchon 314 et introduction de liquide (non représenté), par exemple du sang, dans le compartiment 302, à travers son ouverture supérieure. La figure 7C est une vue de détails agrandie d'une partie de la figure 7B. On observe, en figures 7B et 7C, que l'embout 304 est maintenu en position dans une pièce 322 et qu'un joint torique 324 assure l'étanchéité de la liaison entre l'intérieur de l'embout et donc, à travers le filtre 308, l'intérieur du compartiment 302 et la chambre d'aspiration du système d'aspiration. On observe que l'embout 304 dépasse à l'intérieur de la chambre d'aspiration.

Ainsi, on filtre, par aspiration dans la chambre de dépression, les cellules plus petites que les cellules d'intérêt et les éventuelles cellules lysées et le principal du liquide présent dans le compartiment 302.

Comme illustré en figure 7D, après filtration, on retire le dispositif de la platine. Puis, comme illustré en figure 7E et 7F, on retire l'embout 304 après une rotation le libérant des ergots 318. Puis, comme illustré en figures 7G, 7H et 7I, on insère l'extrémité du compartiment 302 dans un support de tube Eppendorf 328 muni d'un tube Eppendorf 330.

Comme illustré en figures 7J et 7K, le moyen mobile 310 est ensuite mis en mouvement vers le bas, parallèlement à l'axe du compartiment 302. Lors de ce mouvement, les pattes 316 du moyen mobile 310 mis en mouvement par les doigts d'un opérateur, exercent une force verticale, de haut en bas, sur le support de filtre 308 et le libère de l'extrémité inférieure du compartiment 302. Le support de filtre 308 s'enfonce alors dans le tube Eppendorf 330.

Enfin, comme illustré en figure 7L, on retire le compartiment 302 et le moyen mobile 310.

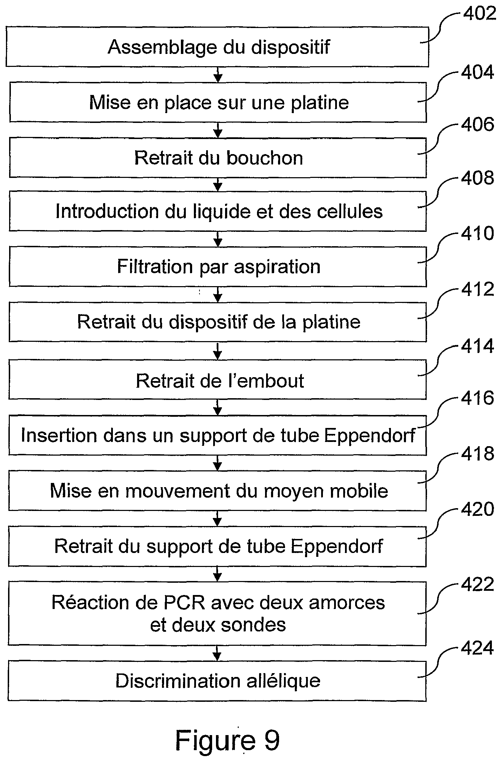

La figure 9 récapitule les étapes ainsi mises en oeuvre.

Au cours d'une étape 402, on assemble les pièces du dispositif. Au cours d'une étape 404, on met en place le dispositif dans une platine 320 d'un système d'aspiration de type connu (non représenté). Au cours d'une étape 406, on retire le bouchon 314. Au cours d'une étape 408, on introduit un liquide, par exemple, du sang, contenant des cellules à cultiver, par l'extrémité supérieure du compartiment 302.

Au cours d'une étape 410, on effectue une filtration, par aspiration dans la chambre de dépression, des cellules plus petites que les cellules d'intérêt et les éventuelles cellules lysées et le principal du liquide présent dans le compartiment 302.

Au cours d'une étape 412, on retire le dispositif de la platine. Au cours d'une étape 414, on retire l'embout 304. Au cours d'une étape 416, on insère l'extrémité du compartiment 302 dans un support de tube Eppendorf.

Au cours d'une étape 418, on met en mouvement respectif le moyen mobile et le compartiment pour exercer une force sur le support de filtre et le libérer afin qu'il s'enfonce dans le tube Eppendorf. Enfin, au cours d'une étape 420, on retire le compartiment 302 et le moyen mobile 310 et on referme le bouchon du tube Eppendorf.

La mise en oeuvre du tube Eppendorf est ensuite réalisée de manière connue en soi, par exemple avec des étapes de lyse, de centrifugation et de récupération du matériel génétique avec pré-amplification du génome global.

Au cours d'étapes 422 et 424, on effectue une analyse du matériel génétique, notamment l'ADN ou l'ARN des cellules recherchées, recueilli dans ces tubes 125 ou 126. L'ADN amplifié est utilisé comme matrice pour détecter les mutations de sensibilité ou de résistance aux thérapies ciblées. De plus, ou alternativement, l'ADNc (en anglais « cDNA », « c » signifiant « complémentaire ») provenant de l'ARN par conversion par RT et amplifié est utilisé comme matrice pour détecter le niveau d'expression de gènes de sensibilité ou de résistance aux thérapies ciblées.

Un volume défini du matériel génétique amplifié, notamment l'ADN, est prélevé pour détecter les mutations de sensibilité ou de résistance aux thérapies ciblées à l'aide de couples d'amorces (en anglais « primers ») sens et anti-sens et de couples de sondes et au cours d'une PCR (acronyme de « polymerase chain reaction ») quantitative et en temps réel.

Le principe de la recherche de mutations de sensibilité ou de résistance aux thérapies ciblées mis en oeuvre dans le mode de réalisation représenté est le suivant. La discrimination allélique ou « SNP genotyping assay » (SNP étant l'acronyme de « single nucleotide polymorphism » pour polymorphisme nucléotide simple) permet de renseigner sur la présence ou l'absence d'une mutation ponctuelle au niveau d'un gène. La première étape, 422, d'une discrimination allélique est une réaction de PCR quantitative en temps réel réalisée avec deux amorces pour amplifier la séquence d'intérêt et deux sondes, par exemple de type TaqMan (marque déposée). L'une des sondes reconnaît la séquence mutée et l'autre reconnaît la séquence normale. Les deux sondes sont associées à des fluorochromes différents, par exemple « VIC » pour la sonde s'hybridant à la séquence normale et « FAM » pour la sonde s'hybridant à la séquence mutée. La seconde étape, 424, fait appel à un programme de discrimination allélique mesurant la fluorescence initiale et la fluorescence finale émise par les fluorochromes FAM ou/et VIC. Ce programme permet de faire la distinction entre les différentes séquences présentes dans chaque échantillon :
- une augmentation de la fluorescence uniquement en VIC indique un profil homozygote pour la séquence normale,
- une augmentation de la fluorescence uniquement en FAM indique un profil homozygote pour la séquence mutée,
- une augmentation de la fluorescence à la fois en VIC et en FAM indique un profil hétérozygote.

Pour la détection de mutations, on met en oeuvre, par exemple, les séquences des amorces et sondes suivantes (sens et anti-sens, 5' à 3') :
Pour la mutation G12D du gène K-ras de résistance à l'Erlotinib et au Gefitinib :
   - amorce sens (ou « forward ») AGGCCTGCTGAAAATGACTGAATAT,
   - amorce anti-sens (ou « reverse ») TCGTCCACAAAATGATTCTGAATTAGCT,
   - sondes « sauvage », couleur « VIC » TTGGAGCTGGTGGCGT,
   - sonde « muté », couleur « FAM » TGGAGCTGATGGCGT.

Pour la mutation G12V (codant « 12 ») du gène K-ras de résistance à l'Erlotinib et au Gefitinib :
- amorce sens (ou « forward ») AGGCCTGCTGAAAATGACTGAATAT,
- amorce anti-sens (ou « reverse ») TCGTCCACAAAATGATTCTGAATTAGCT,
- sondes « sauvage », couleur « VIC » TTGGAGCTGGTGGCGT,
- sonde « muté », couleur « FAM » TTGGAGCTGTTGGCGT.

Pour la mutation G13C du gène K-ras de résistance à l'Erlotinib et au Gefitinib :
- amorce sens (ou « forward ») AGGCCTGCTGAAAATGACTGAATAT,
- amorce anti-sens (ou « reverse ») TCGTCCACAAAATGATTCTGAATTAGCT,
- sondes « sauvage », couleur « VIC » TTGGAGCTGGTGGCGT,
- sonde « muté », couleur « FAM » TTGGAGCTGGTTGCGT.

Pour la mutation L858R du gène EGFR de sensibilité augmentée à l'Erlotinib et au Gefitinib :
- amorce sens (ou « forward ») GCAGCATGTCAAGATCACAGATTT,
- amorce anti-sens (ou « reverse ») CCTCCTTCTGCATGGTATTCTTTCT,
- sondes « sauvage », couleur « VIC » CAGTTTGGCCAGCCCA,
- sonde « muté », couleur « FAM » CAGTTTGGCCCGCCCA.

La signification de « sens » et « anti-sens », et « 5' à 3' » est bien connue de l'homme du métier. Les sondes s'apparient entre les deux amorces et révèlent la présence ou l'absence d'une mutation du fait de leur couleur de fluorescence associée. Pour mémoire, la couleur « FAM » est dans les bleus et la couleur « VIC » est dans les verts. Une mesure de l'intensité de la fluorescence dans chacune de ces couleurs, par l'appareil de PCR, permet de discriminer les gènes normaux, les gènes homozygotes mutés et les gènes hétérozygotes mutés. Par exemple, on réalise 50 cycles d'amplification.

Dans d'autres modes de réalisation, un volume défini du matériel génétique notamment l'ARN converti en ADNc par RT (acronyme de « reverse transcription ») et amplifié est prélevé pour détecter le niveau d'expression de gène de sensibilité ou de résistance aux thérapies ciblées à l'aide de couples d'amorces sens et anti-sens et d'une sonde et au cours d'une PCR (acronyme de « polymerase chain reaction ») quantitative et en temps réel, par exemple avec 50 cycles.

Dans chacun des modes de réalisation décrits ci-dessus, préférentiellement, le filtre est réalisé en polycarbonate avec un traitement de surface hydrophile. L'utilisation d'un tel filtre améliore le taux de retenu des cellules particulières et réduit l'adhérence des autres cellules ou de leur contenu, lorsqu'elles ont été spécifiquement lysées.

Le filtre présente, préférentiellement, un diamètre de pores centré sur une valeur inférieure, par exemple de 1 µm, à la valeur correspondante mise en oeuvre pour les mêmes cellules fixées, c'est-à-dire rendues rigides.

Par exemple, si, pour les cellules fixées, le diamètre des pores aurait été centré sur 7,5 µm, il est ici centré sur une valeur inférieure, par exemple de 6,5 µm. Du fait de la dispersion des diamètres, pratiquement aucun pore ne possède alors un diamètre supérieur à 7 µm.

Pour une application de l'invention à des cellules sanguines, le filtre présente des pores dont la densité est 50.000 et 200.000 pores/cm² et, préférentiellement d'environ 100.000 pores/cm².
Grâce à l'usage d'un filtre en polycarbonate, la dépression nécessaire est beaucoup plus limitée que dans les systèmes de l'art antérieur, jusqu'à être quatre fois moindre, ce qui évite de détériorer les cellules que l'on vise à recueillir sur le filtre. Préférentiellement, une aspiration par dépression inférieure ou égale à -25 mBar est réalisée en dessous du filtre.

Dans des variantes, le support 108 ou 308 de filtre est mécaniquement lié audit moyen mobile jusqu'à l'application, par mouvement du compartiment vers le puits de culture ou le tube Eppendorf, de la force qui libère le support de filtre du moyen mobile. L'inversion des rôles de l'extrémité inférieure du compartiment 102 ou 302 et du moyen mobile 110 ou 310 pour que ce soit ce dernier qui tienne le support de filtre dans ou devant le puits de culture ou le tube Eppendorf et le premier qui le libère lors d'un appui sur l'extrémité supérieure du compartiment est une adaptation aisée, à la portée de l'homme du métier à partir de la description des modes de réalisation donnée ci-dessus.

On observe que ce qui a été exposé ci-dessus pour un seul compartiment 102 ou 302 est, préférentiellement, effectuée simultanément pour un grand nombre de compartiments retenus ensemble par un support commun (non représenté).

Dans des modes de réalisation, la présentation du dispositif objet de la présente invention prend la forme d'un kit comportant, dans un sachet externe, deux sachets internes dont :
- le premier comporte le dispositif monté, tel qu'illustré en figures 2A ou 6A et
- le second comporte le puits de culture et une lame circulaire et/ou un tube Eppendorf ou tout autre accessoire utile à la mise en oeuvre du dispositif.

La mise en oeuvre de la présente invention permet d'éviter des prélèvements risqués de cellules, par exemple de cellules du liquide amniotiques tout en permettant des cultures, par exemple pour une amniocentèse. De plus, du fait de la forme en réservoir du support 108 du filtre, on peut réaliser directement dans ce support des réactions d'immunocytochimie ou d'hybridation fluorescente in situ (« FISH »).

Dans des modes de réalisation (non représentés), une bague est insérée dans l'ouverture inférieure du compartiment en forme de seringue et serrée ou collée dans ce compartiment. Un support de filtre, ou cupule, en forme de rondelle ou bague, est placée dans la bague et retient le filtre. Le filtre est micro perforé et soudé sous la cupule puis inséré avec elle dans l'extrémité inférieure du compartiment en forme de seringue.

A l'intérieur de la partie inférieure du compartiment est positionnée une pièce de forme intérieure conique orientée vers le bas. Cette pièce est en appui sur la surface supérieure du support du filtre. La moitié inférieure de la pièce possède une longueur supérieure ou égale à celle de la bague. La liaison entre le compartiment et la pièce présente ainsi un jeu le long de l'axe du compartiment au moins égal à la distance séparant le haut du support de l'ouverture inférieure de la bague. De cette manière, lorsque la pièce est mise en mouvement axial par un piston inséré dans le compartiment en forme de seringue, elle pousse le support jusqu'à ce qu'il soit libéré de la bague et, par conséquent, du compartiment en forme de seringue. De plus, lorsque l'on insère un piston dans l'ouverture supérieure du compartiment et que l'on appuie sur la partie haute du piston jusqu'à ce qu'il vienne en appui sur la pièce conique, le piston laisse passer l'air entre sa paroi externe et la paroi interne du compartiment et son déplacement n'augmente donc pas la pression à l'intérieur du compartiment, ce qui évite un risque d'endommager les cellules vivantes portées par le filtre. On observe que, grâce à la forme conique de la pièce, le piston n'appui pas sur le filtre et ne risque donc pas d'endommager les cellules retenues sur ce filtre. De même, la pièce n'appuie que sur le support du filtre et ne risque pas, non plus, d'endommager ces cellules.

En poursuivant l'appui sur la partie haute du piston afin qu'il déplace la pièce vers le bas, en mettant en oeuvre le jeu, la pièce fait sortir le support de la bague et du compartiment. Ce support et le filtre tombent alors dans le puits de plaque et/ou le flacon de culture. Préférentiellement la course du piston dans le compartiment, qui est limitée par un épaulement supérieur du piston, est telle qu'en fin de course, lorsque cet épaulement est en appui sur l'ouverture supérieure du compartiment, le jeu a entièrement été utilisé par le déplacement de la pièce. On évite ainsi un risque d'arrachement de la bague qui risquerait de tomber sur le filtre, dans le puits de plaque et/ou le flacon de culture.

## Revendications

1. Dispositif pour isoler des cellules vivantes sur un filtre, **caractérisé en ce qu'**il comporte :
- un support (108, 308) de filtre solidaire d'un filtre, le support de filtre prend la forme d'une rondelle,
- un compartiment (102,302) présentant une ouverture supérieure et une ouverture inférieure adaptée à retenir ledit support et ledit filtre, et
- un moyen mobile (110, 310) par rapport audit compartiment pour appliquer une force sur le support et libérer ledit support, ledit moyen étant adapté à se déplacer parallèlement à l'axe du compartiment pour libérer le support de l'extrémité inférieure du compartiment.

2. Dispositif selon la revendication 1, **caractérisé en ce qu'**il comporte un embout (104, 304) amovible fixé de façon étanche et amovible et adapté à interdire le mouvement relatif du moyen mobile (110, 310) et dudit compartiment (102, 302) qui permet d'appliquer ladite force et libérer ledit support (108, 308) de filtre.

3. Dispositif selon la revendication 2, **caractérisé en ce que** ledit embout (104, 304) présente une ouverture inférieure qui s'adapte à une chambre de dépression d'un système d'aspiration.

4. Dispositif selon la revendication 1 **caractérisé en ce que** ledit support (108) de filtre est en PVC.

5. Dispositif selon la revendication 4, **caractérisé en ce que** ledit support (108) de filtre est en PVC rigide.

6. Dispositif selon l'une quelconque des revendications 1 à 5, **caractérisé en ce que** ledit support (108) de filtre prend la forme d'une lamelle couvre objet.

7. Dispositif selon l'une quelconque des revendications 1 à 6, **caractérisé en ce que** ledit support (108) de filtre a des dimensions supérieures ou égales à une lamelle couvre objet.

8. Dispositif selon l'une quelconque des revendications 1 à 7, **caractérisé en ce que** ledit support (108) de filtre possède une épaisseur inférieure ou égale à 0,4mm et, préférentiellement, à 0,3mm.

9. Dispositif selon l'une quelconque des revendications 1 à 3, **caractérisé en ce que**, ledit support (308) de filtre prend la forme d'une partie, au moins, d'un tube Eppendorf (330).

10. Dispositif selon la revendication 9, **caractérisé en ce que** ledit support (308) de filtre prend la forme d'une partie, au moins, d'un tube Eppendorf (330).

11. Dispositif selon l'une quelconque des revendications 9 ou 10, **caractérisé en ce que** ledit support (308) de filtre reproduit, dans sa partie inférieure intérieure, la forme de la partie inférieure intérieure d'un tube Eppendorf (330).

12. Dispositif selon l'une quelconque des revendications 9 à 11, **caractérisé en ce que** ledit support (308) de filtre reproduit, dans sa partie supérieure extérieure, la forme de la partie supérieure intérieure d'un tube Eppendorf (330).

13. Dispositif selon l'une quelconque des revendications 9 à 12, **caractérisé en ce que** le support (308) de filtre comporte du polycarbonate.

14. Dispositif selon l'une quelconque des revendications 1 à 13, **caractérisé en ce que** le support (108, 308) de filtre est mécaniquement lié audit compartiment (102, 302) jusqu'à l'application de ladite force.

15. Dispositif selon l'une quelconque des revendications 1 à 13, **caractérisé en ce que** ledit support (108, 308) de filtre est mécaniquement lié audit moyen mobile jusqu'à l'application de ladite force.

16. Procédé pour isoler des cellules vivantes sur un filtre ou extraire leur matériel génétique, **caractérisé en ce qu'**il comporte :
- une étape de solidarisation d'un support de filtre solidaire d'un filtre, le support de filtre prenant la forme d'une rondelle, avec un compartiment présentant une ouverture supérieure et une ouverture inférieure et avec un moyen mobile par rapport audit compartiment et
- une étape de mise en mouvement respectif dudit moyen mobile et dudit compartiment pour appliquer une force sur le support de filtre et libérer ledit support de filtre, ledit mouvement se faisant parallèlement à l'axe du compartiment pour appliquer une force sur le support de filtre et libérer ledit support de filtre de l'extrémité inférieure du compartiment.

17. Procédé selon la revendication 16, **caractérisé en ce que**, au cours de l'étape de mise en mouvement, on fait passer directement le support (108) de filtre à un puits de culture (130).

## Patentansprüche

1. Vorrichtung zur Isolierung lebender Zellen auf einem Filter, **dadurch gekennzeichnet, dass** sie Folgendes umfasst:
- eine Filterhalterung (108, 308), die fest mit einem Filter verbunden ist, wobei die Filterhalterung die Form einer Scheibe annimmt,
- ein Fach (102, 302), das eine obere Öffnung und eine untere Öffnung aufweist, die ausgeführt ist, um die besagte Halterung und den besagten Filter zu halten, und
- ein im Verhältnis zu besagten Fach bewegliches Mittel (110, 310) zum Anlegen einer Kraft auf die Halterung und zum Freigeben der besagten Halterung, wobei das besagte Mittel ausgeführt ist, um sich parallel zur Achse des Faches zu bewegen, um die Halterung vom unteren Ende des Faches freizugeben.

2. Vorrichtung nach Anspruch 1, **dadurch gekennzeichnet, dass** sie ein abnehmbares Endstück (104, 304) umfasst, das in abgedichteter und abnehmbarer Form befestigt ist und ausgeführt ist, um die Bewegung des beweglichen Mittels (110, 310) und des besagten Faches (102, 302) zueinander zu unterbinden, das es ermöglicht, die besagte Kraft anzulegen und die besagte Filterhalterung (108, 308) freizugeben.

3. Vorrichtung nach Anspruch 2, **dadurch gekennzeichnet, dass** das besagte Endstück (104, 304) eine untere Öffnung aufweist, die sich einer Vakuumkammer eines Saugsystems anpasst.

4. Vorrichtung nach Anspruch 1, **dadurch gekennzeichnet, dass** die besagte Filterhalterung (108) aus PVC gefertigt ist.

5. Vorrichtung nach Anspruch 4, **dadurch gekennzeichnet, dass** die besagte Filterhalterung (108) aus hartem PVC gefertigt ist.

6. Vorrichtung nach irgendeinem der Ansprüche 1 bis 5, **dadurch gekennzeichnet, dass** die besagte Filterhalterung (108) die Form eines Objektträgers annimmt.

7. Vorrichtung nach irgendeinem der Ansprüche 1 bis 6, **dadurch gekennzeichnet, dass** die besagte Filterhalterung (108) Abmessungen größer oder gleich einem Objektträger aufweist.

8. Vorrichtung nach irgendeinem der Ansprüche 1 bis 7, **dadurch gekennzeichnet, dass** die besagte Filterhalterung (108) eine Dicke kleiner oder gleich 0,4 mm, und vorzugsweise 0,3 mm aufweist.

9. Vorrichtung nach irgendeinem der Ansprüche 1 bis 3, **dadurch gekennzeichnet, dass** die besagte Filterhalterung (308) die Form zumindest eines Teils eines Eppendorf-Reaktionsgefäßes (330) annimmt.

10. Vorrichtung nach Anspruch 9, **dadurch gekennzeichnet, dass** die besagte Filterhalterung (308) die Form zumindest eines Teils eines Eppendorf-Reaktionsgefäßes (330) annimmt.

11. Vorrichtung nach irgendeinem der Ansprüche 9 oder 10, **dadurch gekennzeichnet, dass** die besagte Filterhalterung (308) in ihrem unteren inneren Abschnitt die Form des inneren unteren Abschnitts eines Eppendorf-Reaktionsgefäßes (330) reproduziert.

12. Vorrichtung nach irgendeinem der Ansprüche 9 bis 11, **dadurch gekennzeichnet, dass** die besagte Filterhalterung (308) in ihrem oberen äußeren Abschnitt die Form des oberen äußeren Abschnitts eines Eppendorf-Reaktionsgefäßes (330) reproduziert.

13. Vorrichtung nach irgendeinem der Ansprüche 9 bis 12, **dadurch gekennzeichnet, dass** die besagte Filterhalterung (308) Polycarbonat umfasst.

14. Vorrichtung nach irgendeinem der Ansprüche 1 bis 13, **dadurch gekennzeichnet, dass** die Filterhalterung (108, 308) bis zum Anlegen der besagten Kraft mechanisch mit dem besagten Fach (102, 302) verbunden ist.

15. Vorrichtung nach irgendeinem der Ansprüche 1 bis 13, **dadurch gekennzeichnet, dass** die besagte Filterhalterung (108, 308) bis zum Anlegen der besagten Kraft mechanisch mit dem besagten beweglichen Mittel verbunden ist.

16. Verfahren zur Isolierung lebender Zellen auf einem Filter oder Extrahieren ihres genetischen Materials, **dadurch gekennzeichnet, dass** es Folgendes umfasst:
- einen Schritt zur festen Verbindung einer Filterhalterung, die fest mit einem Filter verbunden ist, wobei die Filterhalterung die Form einer Scheibe annimmt, mit einem Fach, das eine obere Öffnung und eine untere Öffnung aufweist, und mit einem Mittel, das im Verhältnis zum besagten Fach beweglich ist, und
- einen Schritt zum in Bewegung setzen des besagten beweglichen Mittels und des besagten Faches zueinander, um eine Kraft an der Filterhalterung anzulegen und die besagte Filterhalterung freizugeben, wobei die besagte Bewegung parallel zur Achse des Faches erfolgt, um eine Kraft an der Filterhalterung anzulegen und die besagte Filterhalterung vom unteren Ende des Faches freizugeben.

17. Verfahren nach Anspruch 16, **dadurch gekennzeichnet, dass** man im Laufe des Schrittes zum in Bewegung setzen die Filterhalterung (108) direkt zu einer Kulturvertiefung (130) führt.

## Claims

1. Device for isolating living cells on a filter, **characterized in that** it comprises:
- a filter mount (108, 308) attached to a filter, the filter mount taking the form of a washer;
- a compartment (102, 302) having an upper opening and a lower opening designed to hold said mount and said filter; and
- a means (110, 310), mobile in relation to said compartment, for exerting a force on the mount and releasing said mount, said means being designed to move parallel to the axis of the compartment to release the mount from the lower extremity of the compartment.

2. Device according to claim 1, **characterized in that** it comprises a removable end-fitting (104, 304) fixed in a hermetic and removable way, and designed to prohibit the relative movement of the mobile means (110, 310) and said compartment (102, 302), which makes it possible to exert said force and release said filter mount (108, 308).

3. Device according to claim 2, **characterized in that** said end-fitting (104, 304) has a lower opening which adjusts to fit a vacuum chamber of an aspiration system.

4. Device according to claim 1, **characterized in that** said filter mount (108) is made of PVC.

5. Device according to claim 4, **characterized in that** said filter mount (108) is made of rigid PVC.

6. Device according to any one of claims 1 to 5, **characterized in that** said filter mount (108) takes the form of a cover-glass.

7. Device according to any one of claims 1 to 6, **characterized in that** the dimensions of said filter mount (108) are greater than or equal to those of a cover-glass.

8. Device according to any one of claims 1 to 7, **characterized in that** the thickness of said filter mount (108) is less than or equal to 0.4mm, preferably 0.3mm.

9. Device according to any one of claims 1 to 3, **characterized in that** said filter mount (308) takes the form of one portion, at least, of an Eppendorf tube (330).

10. Device according to claim 9, **characterized in that** said filter mount (308) takes the form of one portion, at least, of an Eppendorf tube (330).

11. Device according to any one of claims 9 or 10, **characterized in that** said filter mount (308) reproduces, in its inner lower portion, the form of the inner lower portion of an Eppendorf tube (330).

12. Device according to any one of claims 9 to 11, **characterized in that** said filter mount (308) reproduces, in its upper outer portion, the form of the upper inner portion of an Eppendorf tube (330).

13. Device according to any one of claims 9 to 12, **characterized in that** the filter mount (308) comprises polycarbonate.

14. Device according to any one of claims 1 to 13, **characterized in that** the filter mount (108, 308) is mechanically connected to said compartment (102, 302) until said force is exerted.

15. Device according to any one of claims 1 to 13, **characterized in that** said filter mount (108, 308) is mechanically connected to said mobile means until said force is exerted.

16. Method for isolating living cells on a filter, or for extracting their genetic material, **characterized in that** it comprises:
- a step of coupling a filter mount attached to a filter, the filter mount taking the form of a washer; with a compartment having an upper opening and a lower opening, and with a means mobile in relation to said compartment; and
- a step of putting said mobile means and said compartment in relative motion to exert a force on the filter mount and release said filter mount, said movement occurring parallel to the axis of the compartment to exert a force on the filter mount and release said filter mount from the lower extremity of the compartment.

17. Method according to claim 16, **characterized in that**, during the moving step, the filter mount (108) is moved directly to a culture well (130).
